# EUROPEAN PATENT APPLICATION

(11) **EP 2 388 019 A1**
(43) Date of publication of application: **23.11.2011**
(21) Application number: 09838421.7
(22) Date of filing: 02.02.2009
(51) Int. Cl.: A61L 27/14

(54) **BONE-REGENERATING COMPOSITION CONTAINING ANGIOGENIN**

(30) Priority: 16.01.2009 KR 20090003889
(71) Applicant: Industry-Academic Cooperation Foundation Wonkwang University, Jellabuk-do 570-749 (KR)
(72) Inventor: LEE, Jun, Daejeon-si 305-755 (KR); YOON, Dong-Hyeon, Gumi-si Gyeongsangbuk-do 730-040 (KR); HWANG, Dong-Hyeon, Jeonju-si Jeollabuk-do 560-842 (KR)
(74) Representative: Lecca, Patricia S.
(86) International application number: PCT/KR2009/000500
(87) International publication number: WO 2010/082700

(57) **Abstract**

The present invention relates to a bone-regenerating composition containing angiogenin and to a bone-generating scaffold comprising the composition. Angiogenin has a superior ability to induce initial angiogenesis and bone regeneration as compared to the platelet rich plasma (PRP) which is used as a conventional element for stimulating bone regeneration, thus improving the speed of bone regeneration

## Description

### [Technical Field]

The present invention relates to a composition for bone regeneration containing angiogenin and a scaffold for bone regeneration including the composition.

### [Background Art]

Aesthetic reconstruction of the maxillofacial loss caused by the bone defects is an important challenge in dental diseases. Great efforts have been made to overcome the above-described problem, and such efforts have been associated with bone graft materials, osteoinductive materials, tissue engineering, etc.

Cells, scaffolds, and cytokines are maintained under adequate environments and time in a large cast designed to form a bone, thereby forming a high-quality structure. The previous research has focused in the development of a marketable scaffold, and a large number of materials such as autogenous bone, allogeneic bone, xenogeneic bone, synthetic bone, etc. have been developed and distributed. However, since the harvest of autogenous bone may cause an injury to a patient's body and the amount of autogenous bone harvested is limited, a variety of materials suitable for the osteoconduction have been prepared by modifying the composition of internal components of allogeneic bone or synthetic bone. In recent years, extensive research aimed at enhancing the osteoinduction by isolating platelet-rich plasma, a kind of cytokine, from a patients blood and mixing the platelet-rich plasma with a pre-existing bone has been reported, and positive results have been obtained from the practical application to patients. However, since this approach is not effective, compared to sampling of a patient's blood, other recombinant materials are required.

The bone healing process such as bone graft is developed by complex mechanisms such as migration, differentiation, activation, etc. of various tissues and cells. In these mechanisms, angiogenesis plays an important role in the homeostasis and regeneration of bone tissue. In the 18^{th} and early 20^{th} centuries, the importance of osteoblasts in the bone regeneration has been mainly studied. In 1963, however, Trueta reported that there were angiogenic factors secreted in a fracture area. Since then, angiogenesis in the bone regeneration has been an important subject matter (Trueta J. J. Bone Joint Surg. 45(B):402-418, 1963). A platelet derived growth factor (PDGF), a vascular endothelial growth factor (VEGF), an epidermal growth factor (EGF), and a fibroblast growth factor (FGF) were reported to play an important role in a primary bone formation process to produce a bone matrix (Kanczler JM et al. European cells and Materials 15: 100-114, 2008). Platelet-rich plasma, a currently widely used material, is self-sampled from a patient or extracted by centrifugation and used for bone grafting. In the past research, it was found that platelet was rich in PDGF and TGF-β. While the platelet can be easily handled in the form of gel, it should be immediately used to preserve the activity of growth factors. Moreover, with the rise of tissue engineering, a more effective angiogenic material is required, compared to the conventional methods.

### [Disclosure]

### [Technical Problem]

The present inventor has made a great effort to find a more effective angiogenic material during bone regeneration and confirmed that angiogenin exhibits superior early angiogenesis and bone formation compared to conventional platelet rich plasma (PRT) to promote the bone regeneration rate, thus completing the present invention.

Therefore, an object of the present invention is to provide a composition bone regeneration containing angiogenin.

Moreover, another object of the present invention is to provide a scaffold for bone regeneration including the composition.

### [Technical Solution]

Angiogenin is a polypeptide that is involved in angiogenesis. In the present invention, angiogenin may be derived from a mammal, preferably a human. Preferably, the angiogenin may be derived from the same subject to be treated with angiogenin. For example, angiogenin set forth in SEQ ID NO: 1 may be used.

In the present invention, it is preferred that the angiogenin be produced based on recombinant DNA technology.

In one aspect, the angiogenin may be produced by (a) inserting a DNA sequence coding for angiogenin into a vector including at least one expression control sequence, the vector being operationally connected to the DNA sequence to control the expression of the angiogenin, (b) transforming a host with the resulting recombinant expression vector, (c) culturing the resulting transformant in a suitable medium under suitable conditions to express the DNA sequence, and (d) isolating the angiogenin from the culture medium.

The term "vector," refers to a DNA construct containing a DNA sequence operationally connected to a suitable control sequence to express the DNA sequence in a suitable host. The vector may be a plasmid, a phage particle or simply a potential genomic insert.

The "control sequence" means a nucleic acid sequence that is essential or advantageous for the expression of angiogenin. The control sequence includes a promoter, an upstream activating sequence, an enhancer, a polyadenylation sequence, a transcription terminator, etc.

The "host cell" includes know eukaryotic and prokaryotic hosts such as *Escherichia coli* (E. *coli*)*, Pseudomonas* sp., *Bacillus* sp., *Streptomyces* sp., fungus, and yeast, insect cells such as *Spodoptera frugiperda,* animal cells such as CHO and mouse cells, tissue-cultured human and plant cells, etc.

The transformation and transfection may be performed according to the method as described in the basic experimental procedure (Davis et al. Basic Methods in Molecular Biology, 1986). Preferred examples of the method may include electroporation, transduction, calcium phosphate transfection, cationic lipid-mediated transfection, etc.

Host cells may be cultured in a suitable medium under suitable conditions where an angiogenic protein can be expressed and/or isolated. The cell culturing is performed using a known technique in a suitable nutriment medium containing carbon and nitrogen supply sources and an inorganic salt. A suitable medium is commercially available, and may be prepared from the components and their composition ratio described in the catalogue of the American Type Culture Collection (ATCC), for example.

Angiogenin may be isolated from a culture using a method known in the art. For example, the angiogenin may be isolated from a culture by a method including, but is not limited to, centrifugation, filtration, extraction, spray drying, evaporation, or precipitation. Moreover, the angiogenin may be purified by various methods known in the art such as chromatography or electrophoresis.

Angiogenin may be mixed with a pharmaceutically available carrier according to a typical method. Examples of a suitable carrier may include lactose, dextrose, sucrose, sorbitol, mannitol, xylitol, maltitol, alginate, calcium phosphate, calcium silicate, cellulose, methyl cellulose, amorphous cellulose, polyvinylpyrrolidone, water, methyl hydroxybenzoate, propyl hydroxybenzoate, talc, magnesium stearate, and mineral oil. If necessary, the composition may further include a filler, an anti-coagulating agent, a lubricant, a wetting agent, an emulsifying agent, a preservative, etc.

The composition of the present invention may be formulated by a method well know in the art to provide quick or delayed release of effective components after administration to a subject. The formulation may be in a form of a tablet, a powder, a pill, an emulsion, a solution, a syrup, an aerosol, a soft or hard gelatin capsule, a sterile injection solution, or a sterile powder. Preferably, the formulation may be administered in a dosage form.

The composition bone regeneration containing angiogenin according to the present invention may be grafted separately from a scaffold or together with a scaffold including the composition. Therefore, the present invention relates to a scaffold for bone regeneration including the composition.

The scaffold for bone regeneration includes any of scaffolds for bone regeneration known in the art. The scaffold for bone regeneration may be autogenous bone, allogeneic bone, xenogeneic bone, or synthetic bone. The synthetic bone includes, but is not limited to, hydroxyapatite (HA), collagen, ceramic scaffold, porous calcium phosphate, etc.

In one aspect, the scaffold for bone regeneration may be a scaffold for bone regeneration that has a predetermined concrete shape and includes fibrin glue, bone powder mixed with the fibrin glue, and a plurality of pores formed to accommodate a bone growth promoting factor (angiogenin).

The scaffold according to the present invention is **characterized in that** it has a solid and concrete shape. For this purpose, it is preferred that the fibrin glue be mixed with the bone powder and freeze-dried. Moreover, the scaffold according to the present invention may be treated to have a predetermined shape before being freeze-dried, or may be freeze-dried in a predetermined cast. In one aspect, the shape and the cast may correspond to a jawbone or tooth defect area of a patient. More particularly, the cast may be prepared by (a) preparing a 3-dimensional (3D) mold using 3D CT and (b) preparing a cast for preparation of a scaffold suitable for the bone defect area in the 3D mold using a dental resin.

In the present invention, the term "bone powder" refers to a ground bone powder, preferably a ground bone (inorganic) powder, from which osteoblasts are removed. The bone powder may be derived from at least one selected from the group consisting of autogenous bone, allogeneic bone, xenogeneic bone, and synthetic bone (for example, hydroxyapatite). In the present invention, the bone powder is commercially available from, for example, Dynagraft (Austem Co. Ltd.), Biocera (Oscotec In.), Bio-Oss (Jungsan Biomed Co. Ltd.), ICB (Purgo), MBCP (Purgo), etc.

In the present invention, the term "fibrin glue" refers to a biocompatible and biodegradable product including fibrinogen and thrombin as main components. The fibrin glue has been used in a variety of applications. For example, the fibrin glue has been clinically applied for substitution or reinforcement of sutures by applying fibrinogen, thrombin, calcium chloride, or a fibrinolytic enzyme inhibitor as a tissue adhesive to suture peripheral nerves and fine blood vessels through tissue agglutination of fibrin in Europe. Moreover, in Japan, the fibrin glue has been used as a surgical adhesive for the cerebral nerve surgery including a. vascular surgery field, the orthopedic surgery such as bone adhesion, and the arrest of bleeding in patients suffering from lacerated wound, etc. For example, Greenplast (Green Cross Corp.), Beriplast-P (Aventis), Tisseel (batter), etc. are commercially available.

The fibrin glue according to the present invention preferably includes fibrinogen and thrombin. The fibrinogen may be used in a concentration of 10 to 1000 mg/ml, and preferably 10 to 100 mg/ml. The thrombin may be used in a concentration of 0.1 to 1000 IU/ml, and preferably 1 to 100 IU/ml.

The fibrin glue according to the present invention may further include aprotinin or calcium chloride. Moreover, the fibrin glue according to the present invention may further include a water-soluble binder. The water-soluble binder may be a cell culture medium, distilled water, or blood.

In the present invention, when bone powder and fibrin glue are mixed together, an increase in the amount of the fibrin glue may increase the possibility of causing the toxicity, and therefore it is preferred to suitably adjust the content of the fibrin glue. Moreover, when the size of the scaffold is larger, it is preferred to increase the content of the bone powder to maintain the intensity and shape of the scaffold. In view of these facts, the fibrin glue and the bone powder may be mixed in a volume ratio of 1:1 to 10, preferably 1:1 to 5, and more preferably 1:1 to 3 in the present invention.

The bone growth promoting factor according to the present invention may include a variety of factors for promoting bone growth in addition to the angiogenin. For example, the bone growth promoting factor may include a hormone, a cytokine other than the angiogenin, a stem cell, etc. More particularly, the bone growth promoting factor may be a platelet-derived growth factor (PDGF) or a vascular endothelial growth factor (VEGF).

Since a large number of pores are formed in the mixture of fibrin glue and bone powder while the mixture is subjected to a freeze-drying process, the scaffold according to the present invention can improve absorption and maintenance of the bone growth promoting factor such as angiogenic. The freeze-dried scaffold readily absorbs a medium (or a carrier) containing the bone growth promoting factor and transfers the medium into the pores.

### [Advantageous Effects]

The angiogenin can exhibit superior early angiogenesis and bone formation compared to the conventional platelet rich plasma (PRT) used as a bone regeneration promoting factor, thereby achieving the more rapid bone regeneration.

### [Description of Drawings]

FIG. 1 schematically shows a process of extracting platelet-rich plasma from a miniature pig.
FIG. 2 shows the positions in which a bone graft material according to the present invention is grafted in an alveolus defect area of a miniature pig.
FIG. 3 is a graph illustrating the ratio of bone volumes in an experimental group and a control group.
FIG. 4 is a graph illustrating the ratio of bone surface to bone volume in the experimental group and the control group.
FIG. 5 is a graph illustrating the ratio of bone surface densities in the experimental group and the control group.
FIG. 6 is a graph illustrating the ratio of trabecular thicknesses in the experimental group and the control group.
FIG. 7 is a graph illustrating the ratio of trabecular numbers in the experimental group and the control group.
FIG. 8 shows the histological features of a clot-grafted group in the experimental group and the control group over time.
FIG. 9 shows the histological features of a synthetic bone-grafted, group in the experimental group and the control group over time.
FIG. 10 shows the histological features of an autogenous bone-grafted group in the experimental group and the control group over time.

### [Mode for Invention]

Hereinafter, preferred embodiments of the present invention will be described with reference to the accompanying drawings.

Hereinafter, exemplary embodiments of the present invention will be described in detail below with reference to the accompanying drawings such that those skilled in the art to which the present invention pertains can easily practice the present invention.

### Example 1: Materials and Methods for Experiments

### 1-1. Experimental Animals and Materials

Eight 30 kg male miniature pigs (small and tailored experimental animals, PWGetics, Korea) were used as experimental animals. The pigs were bred with soft animal feed and water at room temperature in certified breeding facilities for a predetermined period of time.

### 1) Tooth Extraction

During the cell culture, teeth from a premolar tooth to a first molar tooth were extracted from the left and right lower jaws of a miniature pig. After the extraction of the teeth, the wounds were continuously sutured. One week after the suturing process, an injection was performed, and the wounds were healed for one month. During the healing process, additional extraction of impacted teeth was performed during surgery.

### 2) Preparation of Graft Materials

### (1) Platelet-Rich Plasma

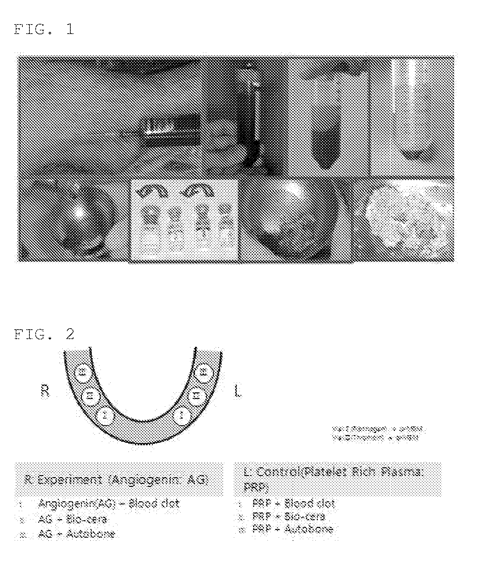

50 cc of venous blood was sampled from a miniature pig, and centrifuged in a typical manner twice to obtain the platelet-rich plasma (FIG. 1).

### (2) Isolation and Purification of Recombinant Angiogenin

pET11a-bovine angiogenin and E. *coli* were inoculated in an LB medium (Trypton : Yeast Extract : NaCl = 2 : 1: 1), and cultured at 37 °C. Then, 0.1 mM isopropyl thio-β-D-galactopyranoside (IPTG) was added to the resulting culture medium and cultured for 2 to 3 hours. Subsequently, E. *coli* was centrifuged (6000 rpm, 4 °C, 15 minutes) and kept at - 70 °C. The freeze-dried E. *coli* was re-suspended in a buffer solution (20 mM Tris-HCl, (pH 7.6, 10 % sucrose containing 2.5 mM PMSF, 100 µg/ml Lysozyme, 200 mM NaCl, 10 mM EDTA), and kept in ice for 45 minutes. Finally, 2.5 mM PMSF was added to the E. *coli* suspension such that E. *coli* was lysed using an ultrasonic processor or a French presser. E. *coli* was centrifuged (17,300 g, 4 °C, 25 minutes) and subjected to SDS-PAGE to confirm the expression of angiogenin. As a result, the angiogenin was expressed in the form of an inclusion body. Then, the inclusion body was refolded. The inclusion body was washed with 20 mM Tris-HCl (pH 7.6), and solubilized in 7 M guanidine-HCl (pH 7.5, containing 100 mM potassium phosphate and 100 mM mercaptoethanol). The solubilized angiogenin was diluted in a 50 mM Tris-HCl solution (pH 8.5) containing 100 mM NaCl. Then, the diluted angiogenin solution was kept at 4 °C for 24 hours and stirred for 6 to 8 hours. Finally, 1 M NaCl was slowly added to the angiogenin solution. Subsequently, the resulting angiogenin sample was concentrated to isolate a recombinant angiogenin using C18 reverse phase HPLC. The recombinant angiogenin had the same amino acid sequence as set forth in SEQ ID NO: 1.

### 1-2. Experimental Methods

### 1) Anesthesia Induction

For bone grafting of a miniature pig, each of an animal anesthetic (Rompun® 3 mg/kg, Bayer Korea Co., Ltd., Korea) and ketamine was intravenously injected to the miniature pig to induce general anesthesia. Oral intubation was performed to induce general anesthesia using N₂O + O₂. In order to provide a field of vision during the surgery, dogteeth of the upper and lower jaws were tied with a bandage to induce a maximum opening degree. The oral cavity was sterilized with a Potadine solution, and 2% lidocaine (Yuhan Co, Ltd., Korea) containing epinephrine in a volume ratio of 1:100,000 was injected into the residual alveolar bone of the lower jaw to induce local anesthesia and stop bleeding.

### 2) Alveolar Bone Exposure

The residual alveolar bone of the miniature pig was subjected to horizontal incision and vertical incision, and the periosteum was peeled off to expose the buccal and lingual sides of the lower jaw to the maximum.

### 3) Formation of Control and Experimental Groups

Round bone defect areas with a depth of 8 mm and a diameter 10 mm were formed using a micro wheel saw having a radius of 3 mm. Rough regions were polished into a desired shape using a surgical round bur or chisel or a mallet. Then, three bone defect areas in each of the left and right lower jaws were formed in the same manner as described above. Autogenous bone required for the experiments was obtained during the formation of the bone defect areas and ground into chip bone using a bone rongeur and a bone mill.

As the control, platelet-rich plasma + clot, platelet-rich plasma + autogenous bone, and platelet-rich plasma + synthetic bone were sequentially inserted into the left bone defect area, and as the experimental group, recombinant angiogenin + clot, recombinant angiogenin + autogenous bone, and recombinant angiogenin + synthetic bone were sequentially inserted into the right bone defect area. Each of the control and experimental groups was injected and fixed as shown FIG. 2.

Releasing incision was performed on the wound to release the tension, and a 4-0 nylon was used to perform a continuous locking suture. Amoxicillin (Tiramox®, SAMJIN Pharmaceutical Co., Ltd., Korea) and diclofenac sodium (Kinpoin®, SAMJIN Pharmaceutical Co., Ltd., Korea) were intramuscularly injected into all experimental animals for three days to prevent infections after surgery, and the experimental animals were fed a soft diet with high-protein milk for one week. Disinfection was performed using chlorhexidine (Hexamedin®, Bukwang Pharmaceutical Co., Ltd., Korea) for one week.

### 1-3. Visual Inspection

After surgery, disinfection of feeds provided to miniature pigs was continuously performed, and the miniature pigs were maintained under healthy conditions. 1, 2, 4 and 8 weeks after the surgery, the miniature pigs were sacrificed, and the healing level of the bone graft area, the inflammatory condition, and the wound dehiscence were observed with the naked eye before the bone tissue samples were immobilize with formalin.

### 1-4. Micro CT Analysis

Block specimens of the experimental areas were imaged using Microfocus X-ray Computed Tomography (µCT, Harmony 130-P3-5, DRGEM co. Korea) under the conditions such as a focal spot size of 5 µm, a field of view of 105 mm, a reconstruction image size of 2048 X 2048 pixels, a depth of reconstruction image of 16 bits, and a detectability of 5 µm, thus obtaining 3D images of the block specimens in 1024 x 1024 x 512 pixels using a cone-beam volumetric reconstruction algorithm. Then, the 3D images were used to quantitatively and qualitatively analyze the difference in bone formation using volume rendering, slab rendering, and 3D measurement technique.

The quantitative analysis was performed in such a manner that the tissue volume, bone volume, and bone surface in each group were measured, and the percent bone volume was calculated by converting the amount of bone occupying in each group into the percent ratio of bone volume to tissue volume based on the measurement results. Moreover, the ratio of bone surface to bone volume in each group was calculated to observe the change. Furthermore, the ratio of bone surface to bone volume in each of the experimental group and the control group was calculated to examine the difference in the two groups.

Moreover, for the qualitative analysis, a bone surface density (1/mm) representing the strength of the bone surface was used to analyze the density of the cortical bone, and the trabecular thickness (mm) and the trabecular number (1/mm) were used to evaluate the bone quality of the cancellous bone and observe the change in trabecular thickness and number, and the ratio between the experimental group and the control group was calculated to examine the difference in the two group.

### 1-5. Histological Inspection

Tissue mass was prepared in the sagittal plane direction, fixed in a 10 % neutral formalin solution for two days, demineralized with 5% nitric acid, and then dehydrated and embedded in resin by a typical method. Then, 4 to 6 µm-sized microtomed samples were attached to a poly-L-lysine-coated slide to prepare samples. In order to examine the changes in shapes of the new bone and its surrounding tissues, the samples were stained with Hematoxylin & Eosin and Masson's trichrome (MT) stains and examined under a microscope.

### 1-6. Histomorphometric Analysis

The stained microtomed samples were imaged using an optimal microscope to obtain images magnified 100 times. 4 and 8 weeks after the bone grafting, three areas were selected from the tissues during secondary bone formation process, and the areas of new bone formation were measured to calculate bone deposition rates (%).

### 1-7. Statistical Analysts

The average and standard deviation (SD) of each group were calculated with the analysis values. Data was subjected to a significance test (p <0.05), followed by an ANOVA test.

### Example 2: Experimental Results

### 2-1. Clinical Features

Neither inflammatory features nor revere bleeding was observed in all the experimental animals, and the wound dehiscence appeared at the beginning in the bone defect areas in the clot-filled group (control). However, there were no specific clinical features in other bone defect areas.

### 2-2. Radiological Features

### Quantitative and Qualitative analyses in Microfocus X-ray Computed Tomography (µCT) Images

**[Table 1]**

| 3D Parameters (Average) of µCT | | | | | | | |
|---|---|---|---|---|---|---|---|
| Parameter | Week | PRP ( Control) | | | Group) | | |
| | | Clot | Biocera | Autobone | Clot | Biocera | Autobone |
| BV/TV (%) | 1 | 5.78 | 15.93 | 19.95 | 7.88 | 25.00 | 33.48 |
| | 2 | 9.54 | 23.47 | 37.22 | 8.43 | 22.11 | 35.85 |
| | 4 | 11.52 | 25.98 | 38.41 | 11.35 | 28.64 | 53.76 |
| | 8 | 23.03 | 43.13 | 52.72 | 28.28 | 51.15 | 72.10 |
| BS/BV (1/Pixel) | 1 | 0.87 | 1.17 | 0.67 | 1.95 | 1.20 | 0.45 |
| | 2 | 1.18 | 1.47 | 0.33 | 1.85 | 0.63 | 1.35 |
| | 4 | 1.71 | 1.13 | 0.61 | 1.86 | 0.65 | 0.54 |
| | 8 | 1.25 | 0.95 | 0.57 | 1.19 | 0.82 | 0.54 |
| Tb.Th (Pixel) | 1 | 5.278 | 4.32 | 9.43 | 3.41 | 5.84 | 8.22 |
| | 2 | 3.810 | 3.94 | 9.38 | 2.82 | 6.16 | 4.04 |
| | 4 | 3.13 | 4.49 | 7.80 | 3.17 | 6.50 | 7.42 |
| | 8 | 4.24 | 5.25 | 8.03 | 4.30 | 5.04 | 6.94 |
| Tb.N (1/Pixel) | 1 | 0.01 | 0.04 | 0.02 | 0.02 | 0.04 | 0.04 |
| | 2 | 0.03 | 0.06 | 0.04 | 0.03 | 0.04 | 0.09 |
| | 4 | 0.04 | 0.06 | 0.05 | 0.04 | 0.04 | 0.07 |
| | 8 | 0.05 | 0.08 | 0.07 | 0.07 | 0.10 | 0.10 |

| | | | | | | | |
|---|---|---|---|---|---|---|---|
| 1) Quantitative analysis ① Percent Bone Volume in Tissues (Bone Volume to Tissue Volume) | | | | | | | |

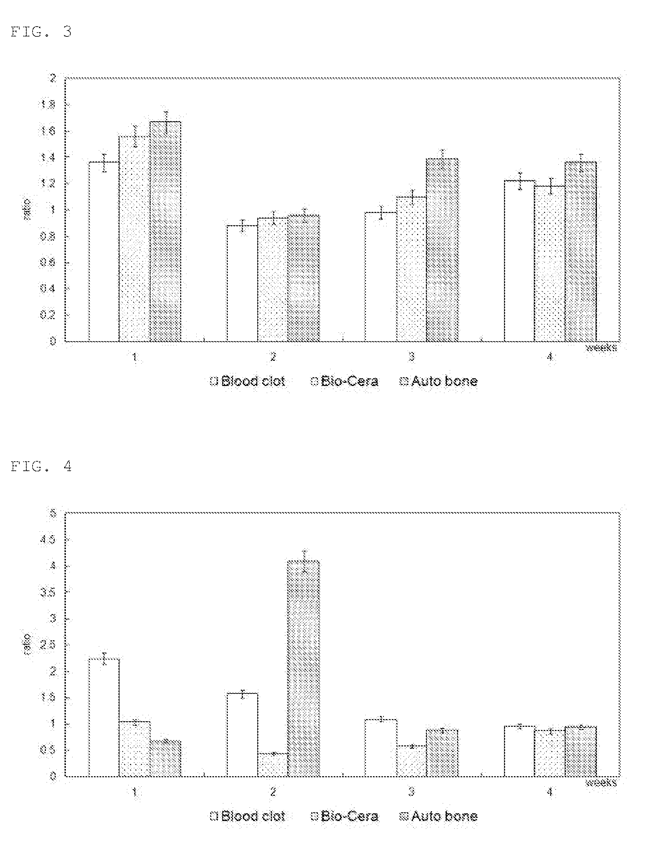

The increase in bone volume was observed over time in both the experimental group and the control group, and the values in the experimental group were higher than those of the control group. In particular, the synthetic bone and autogenous bone of the experimental group were increased to 51.15 and 72.10 at time point of 8 weeks, respectively. The ratio of the clot, synthetic bone, the autogenous bone in the experimental group and the control group was measured at high values of 1.36, 1.56, and 1.67, respectively, at a time point of one week, and the ratio of the autogenous bone was measured at high values of 1.39 and 1.36 at time points of 4 and 8 weeks, respectively. However, no statistically significant difference was observed in each group (FIG. 3, Table 2).

**[Table 2]**

| Ratio of Percent Bone Volumes in Experimental Group and Control Group | | | | |
|---|---|---|---|---|
| Type/Week | 1 | 2 | 4 | 8 |
| Clot | 1.36 | 0.88 | 0.98 | 1.22 |
| Synthetic Bone | 1.56 | 0.94 | 1.10 | 1.18 |
| Autogenous Bone | 1.67 | 0.96 | 1.39 | 1.36 |

| | | | | |
|---|---|---|---|---|
| ② Ratio of Bone Surface to Bone Volume (l/mm) | | | | |

The clotting was increased over time until the time point of 4 weeks in both the groups. In the synthetic bone, the clotting was decreased between one week and two weeks compared to the control group, but continuously increased until the time point of 8 weeks (0.63, 0.65, and 0.82). The ratio of the experimental group to the control group showed a significant difference with a value of 2.24 in the case of the clot at the time point of 1 week and with a value of 4.09 in the case of the autogenous bone at the time point of 2 weeks(p < 0.05) (FIG. 4, Table 3).

**[Table 3]**

| Ratio of Bone Surface to Bone Volume in Experimental Group and Control Group | | | | |
|---|---|---|---|---|
| Type/Week | 1 | 2 | 4 | 8 |
| Clot | 2.24* | 1.57 | 1.09 | 0.95 |
| Synthetic Bone | 1.03 | 0.43 | 0.58 | 0.86 |
| Autogenous Bone | 0.67 | 4.09* | 0.88 | 0.94 |

| | | | | |
|---|---|---|---|---|
| 2) Qualitative Analysis ① Bone Surface Density (l/mm) | | | | |

In the experimental group, the bone surface density of the synthetic bone was decreased to 0.30 and 0.14 at the time points of 1 and 2 weeks, respectively, and the bone surface density of the autogenous bone was decreased to 0.48 and 0.29 at the time points of 2 and 4 weeks, respectively. That is, the increase in the bone surface density was high at these time points.

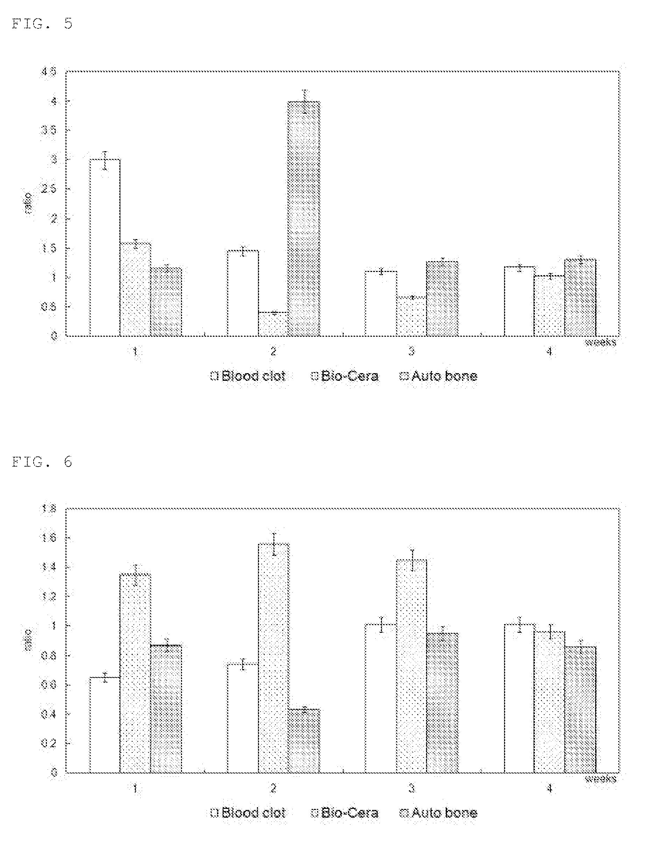

Like the ratio of bone surface to bone volume, the bone surface density in the experimental group and the control group was also high at the time points of 1 and 2 weeks in the case of the clot and the autogenous bone, respectively (FIG. 5, Table 4).

**[Table 4]**

| Ratio of Bone Surface Densities in Experimental Group and Control Group | | | | |
|---|---|---|---|---|
| Type/Week | 1 | 2 | 4 | 8 |
| Clot | 3.00* | 1.45 | 1.10 | 1.17 |
| Synthetic Bone | 1.58 | 0.40 | 0.66 | 1.02 |
| Autogenous Bone | 1.15 | 4.00* | 1.26 | 1.30 |

| | | | | |
|---|---|---|---|---|
| ② Trabecular Thickness (mm) | | | | |

In the experimental group, the trabecular thicknesses were increased at the time points of 2 and 4 weeks, and the trabecular thickness of the autogenous bone was increased to similar values of 7.80 and 7.42 at the time point of 4 weeks in the control group and the experimental group, respectively. The ratio of the trabecular thicknesses in the experimental group and the control group were similar to each other, but the ratio of the trabecular thicknesses of the synthetic bone was relatively high (1.35, 1.56, and 1.45) at the time points of 1, 2 and 4 weeks, respectively. However, there was no statistical significance (FIG. 6, Table 5).

**[Table 5]**

| Ratio of Trabecular Thicknesses in Experimental Group and Control Group | | | | |
|---|---|---|---|---|
| Type/Week. | 1 | 2 | 4 | 8 |
| Clot | 0.65 | 0.74 | 1.01 | 1.01 |
| Synthetic Bone | 1.35 | 1.56 | 1.45 | 0.96 |
| Autogenous Bone | 0.87 | 0.43 | 0.95 | 0.86 |

| | | | | |
|---|---|---|---|---|
| ③ Trabecular Number (l/mm ) | | | | |

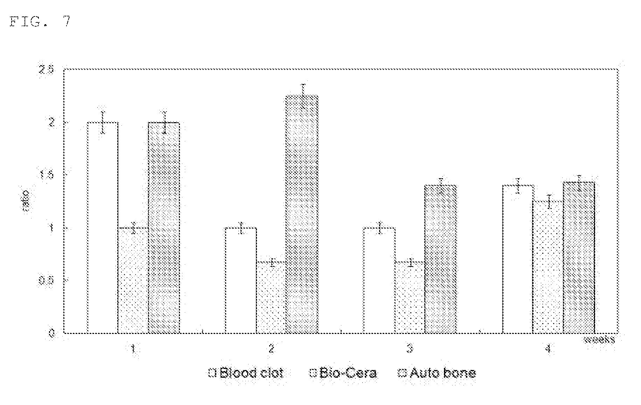

In both the experimental group and the control group, the trabecular number was increased over time. Especially, in the experimental group, the trabecular number of the synthetic bone was highly increased from 0.04 to 0.10 between 4 weeks and 8 weeks. Moreover, the trabecular number of the autogenous bone was highly increased from 0.04 to 0.09 between 1 week and 2 weeks. The ratio of the trabecular numbers in the experimental group and the control group was high with values of 2.00 and 2.00 at the time point of one week in the case of the clot and the autogenous bone. In particular, the ratio of the trabecular numbers was high with a value of 2.25 at the time point of 2 weeks in the case of the autogenous bone (p< 0.05; FIG. 7, Table 6).

**[Table 6]**

| Ratio of Trabecular Numbers in Experimental Group and Control Group | | | | |
|---|---|---|---|---|
| Type/Week. | 1 | 2 | 4 | 8 |
| Clot | 2.00* | 1.00 | 1.00 | 1.40 |
| Synthetic Bone | 1.00 | 0.67 | 0.57 | 1.25 |
| Autogenous Bone | 2.00* | 2.25* | 1.40 | 1.43 |

### 2-3. Histological Features

### 1) Clot-Grafted Group

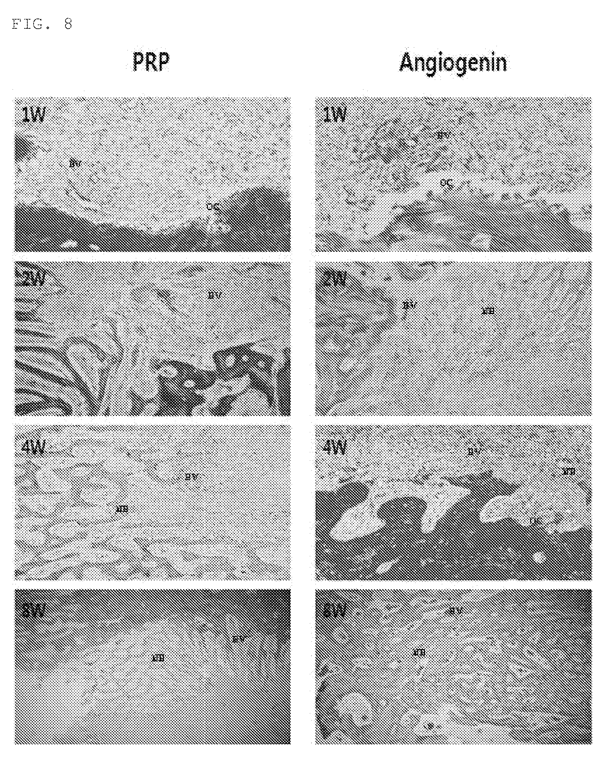

At one week of the PRP experiment, a large amount of bleeding and granulation tissues were observed in the bone defect area, and a moderate level of angiogenesis was observed in the bone defect area. The activity of osteoblasts or the formation of new bone was slightly observed, and the activity of osteoclasts was low (1W on the left side of FIG. 8). At 2 weeks, the angiogenesis was decreased compared to that observe at the one week, but was still on progress, and the activity of the osteoblasts was also observed (2W on the left side of FIG. 8). At 4 weeks, the infiltration of inflammatory cells was significantly decreased, the osteoclasts did not appear, and the osteoconduction in which a new bone was formed was observed around the bone defect area (4W on the left side of FIG. 8). However, the formation of the new bone by the osteoconduction was not observed in the bone defect area, and the new bone was surrounded by the matured fibrous tissue in which the inflammation and new blood vessel were hardly present. At 8 weeks, the inflammation and osteoclasts disappeared from the surroundings of the bone defect area, the activity of the osteoblasts were observed in the bone defect area, and the osteoblasts were substituted with lamella bone (8W on the left side of FIG. 8).

According to the clinical features obtained from the angiogenin-treated clot group at the time point of one week, the infiltration of the inflammatory cells was slightly observed, and the maturation and fibrosis of fibrous connective tissues were significantly observed, compared to the control (1W on the right side of FIG. 8). Moreover, the angiogenesis was active compared to the control, the new bone formation or the activity of the osteoblasts was significantly observed in the bone defect area, unlike the control, and a large amount of osteoclasts were also observed. At 2 and 4 weeks of the experiments, the new bone formation was significantly observed without any of the foreign body reaction and inflammatory reaction compared to the control, and the shape of the fibrous tissue was maintained in the damaged areas (2W and 4W on the right side of FIG. 8). At the time pint of 8 weeks, the foreign body reaction or the infiltration of the inflammatory cells was not observed in the angiogenin-treated clot group, and the new bone formed was almost filled in the bone defect area. Moreover, the bone defect area was substituted with the mature bone compared to the control (8W on the right side of FIG. 8).

### 2) Synthetic Bone-Grafted Group

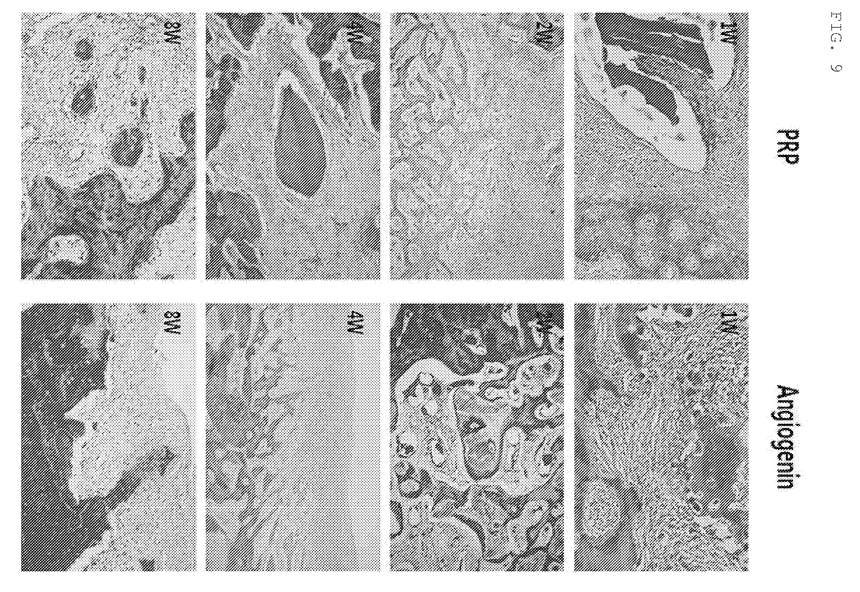

At the time point of one week of the experiment using the synthetic bone in the control, the bleeding and the granulation tissues were slightly observed in the bone defect area compared to the clot group, and the activity of the osteoblasts and the new bone formation were observed at a slightly high level. The activity of the osteoclasts was hardly observed (1W on the left side of FIG. 9). At the time points of 2 and 4 weeks, the infiltration of the inflammatory cells and the fibrous tissue formation were observed at a high level compared to the clot group, and the new bone formation was active (2W on the left side and 4W of FIG. 9). At 8 weeks of the bone grafting, the synthetic bone-grafted area was not completely substituted with the lamella bone, but the rate of osseous fusion was higher than that observed at the time point of 4 weeks (8W on the left side of FIG. 9).

According to the clinical features obtained from the angiogenin-treated group at the time point of one week, the infiltration of the inflammatory cells was slightly observed, and the activity of the osteoclasts was observed at a low level compared to the control. Moreover, the activity of the osteoclasts around the grafted bone was observed at a high level, and the superior fibrosis and new bone formation were observed (1W on the right side of FIG. 9). At 2 and 4 weeks of the experiments, like the control, the new bone formation and the fibrous tissue formation were observed in the damaged areas (2W and 4W on the right side of FIG. 9), and the osseous fusion was almost completely shown at the time point of 8 weeks (2W on the right side of FIG. 9).

### 3) Autogenous Bone-Grafted Group

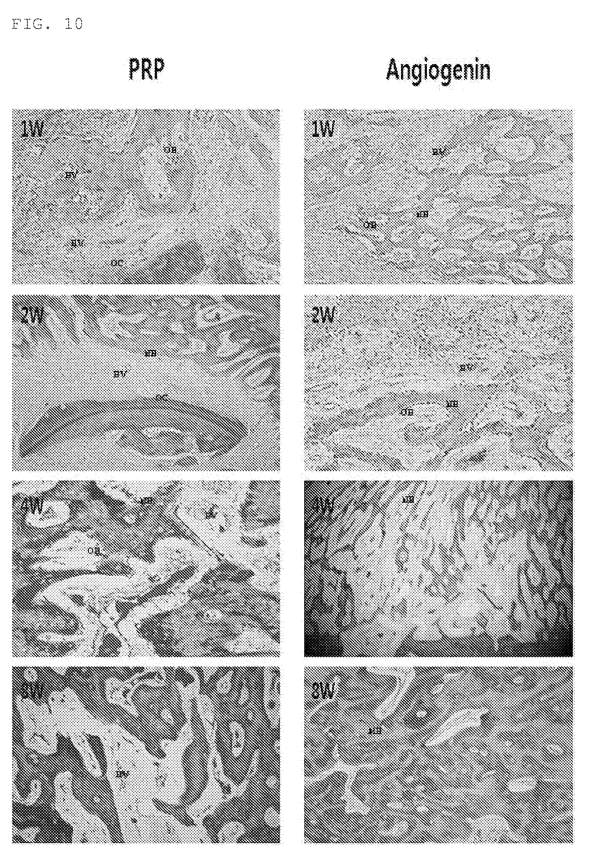

The bleeding and the granulation tissues were hardly observed at the time point of one week in the autogenous bone-grafted group as the control, and the fusion between the host bone and the grafted bone was observed around the bone defect area (1W on the left side of FIG. 10). Moreover, the lowest infiltration of the inflammatory cells was observed, and the new bone formation and the most superior activity of the osteoblasts were observed compared to the synthetic bone- or clot-treated group. Furthermore, the active angiogenesis and new bone formation were observed at the time point of 2 weeks, and the new bone formation was gradually increased at the time point of 4 weeks (2W and 4W on the left side of FIG. 10). The complete osseous fusion was observed in the bone-grafted area at the time point of 8 weeks, and the bone-grafted area was substituted with the lamella bone (8W on the left side of FIG. 10).

In the experimental group, the highest new bone formation was observed at the time point of one week, and the lowest inflammatory reaction was observed the autogenous bone-grafted area of the angiogenin-treated group (1W on the right side of FIG. 10). 2 weeks after the experiments (2W on the right side of FIG. 10), there are no significant difference in the new bone formation, angiogenesis, fibroplasia, and mature bone at the time points of 4 and 8 weeks (4W and 8W on the right side of FIG. 10).

**[Table 7]**

| Histological Analysis Results | | | | | | | |
|---|---|---|---|---|---|---|---|
| | Week | PRP | | | Angiogenin | | |
| | | Clot | Biocera | Autobon e | Clot | Biocera | Autobone |
| Inflammation | 1 | ++ | + | ± | + | + | ± |
| | 2 | + | ± | ± | ± | ± | - |
| | 4 | ± | - | - | - | - | - |
| | 8 | - | + | - | - | - | |
| Hemorrhage | 1 | ++ | ++ | + | + | - | - |
| | 2 | + | ± | ± | - | - | - |
| | 4 | ± | - | - | - | - | - |
| | 8 | - | - | - | - | - | - |
| New bone formation | 1 | ± | + | ++ | + | + | + |
| | 2 | + | ++ | ++ | ++ | ++ | +++ |
| | 4 | + | ++ | +++ | ++ | +++ | +++ |
| | 8 | ++ | ++ | +++ | ++ | +++ | +++ |
| Angiogenesis | 1 | ++ | + | + | +++ | ++ | ++ |
| | 2 | + | ++ | + | ++ | ++ | ++ |
| | 4 | + | + | + | + | + | + |
| | 8 | + | + | ± | + | + | ± |
| Fibrosis | 1 | + | ++ | ++ | ++ | ++ | ++ |
| | 2 | + | + | + | + | + | + |
| | 4 | + | + | + | + | ± | ± |
| | 8 | + | + | + | + | - | ± |

| | | | | | | | |
|---|---|---|---|---|---|---|---|
| (- : negative ± : rare + : mild ++ : moderate +++ : intense) | | | | | | | |

### 2-4. Histomorphometric Features

In the histomorphometric analysis, the superior bone formation in the experimental group was significantly observed at the time point of 2, 4 and 2 weeks in the case of the clot, the synthetic bone, and the autogenous bone, respectively, compared to the control (p < 0.05). Moreover, the areas of the bone formation areas were increased over time in all the groups, but the new bone was subjected to osseous fusion with the host bone at the time point of 8 weeks in the case of the autogenous bone, which showed nearly complete bone maturation.

**[Table 8]**

| Histomorphometric analysis Results (% ± SD) | | | | | | | |
|---|---|---|---|---|---|---|---|
| | Week | PRP | | | Angiogenin | | |
| | | Clot | Biocera | Autobone | Clot | Biocera | Autobone |
| New bone formation | 1 | 7 ±0.04 | 31 ±0.07 | 28 ±0.05 | 10 ±0.05 | 25 ±0.06 | 32 ±0.03 |
| | 2 | 12 ±0.02 | 34 ±0.05 | 33 ±0.07 | 21* ±0.08 | 39 ±0.02 | 43* ±0.05 |
| | 4 | 27 ±0.01 | 39 ±0.07 | 62 ±0.04 | 33 ±0.04 | 47* ±0.02 | 65 ±0.03 |
| | 8 | 57 ±0.07 | 69 ±0.10 | 73 ±0.01 | 62 ±0.02 | 68 ±0.06 | 80* ±0.02 |

| | | | | | | | |
|---|---|---|---|---|---|---|---|
| * P < 0.05, %: New bone area / total grafted area, 3 ROI | | | | | | | |

In this Example, when each of the clot, the synthetic bone, and the autogenous bone was grafted into the bone defect area of the lower jawbone of the miniature pig, the bone defect area was treated with PRP in the control and treated with angiogenin in the experimental group, and fixed with a tissue adhesive. As a result, the visual, radiological, histological, and histomorphometric analyses were performed to determine which effects were obtained during the bone healing process in each group, and the following results were obtained.

As compared to the control, the group in which the miniature pigs were treated with angiogenin exhibited the following results.

First, in the visual inspection, the epithelialization was rapid and the inflammation is low.

Second, in the µCT inspection, the bone volume, the bone density, and the trabecular numbers were observed to be high at the time points of 1 and 2 weeks in the case of the clot and the autogenous bone, respectively.

Third, in the histological and histomorphometric inspections, the new blood vessels were highly proliferated at the beginning of one week, and the bone formation and the ossification were facilitated at the time points of 2 and 4 weeks.

In conclusion, superior early angiogenesis and bone formation were observed in the surgery in which the bone defect area was treated with angiogenin during the bone grafting, compared to the currently used PRP.

### [Industrial Applicability]

According to the present invention, the angiogenin can be used to promote the bone regeneration.

## Claims

1. A composition for bone regeneration comprising angiogenin.

2. The composition according to claim 1, wherein the angiogenin is produced by (a) inserting a DNA sequence coding for angiogenic into a vector including at least one expression control sequence, the vector being operationally connected to the DNA sequence to control the expression of the angiogenin, (b) transforming a host with the resulting recombinant expression vector, (c) culturing the resulting transformant in a suitable medium under suitable conditions to express the DNA sequence, and (d) isolating the angiogenin from the culture medium.

3. The composition according to claim 1, wherein the angiogenin has an amino acid sequence set forth in SEQ ID NO: 1.

4. A scaffold for bone regeneration comprising the composition according to any one of clams 1 to 3.

5. The scaffold according to claim 4, wherein the scaffold comprises fibrin glue, bone powder mixed with the fibrin glue, and a plurality of pores formed to accommodate a bone growth promoting factor, wherein the scaffold has a predetermined concrete shape.

6. The scaffold according to claim 4, wherein the scaffold is treated to have a predetermined shape before being freeze-dried.

7. The scaffold according to claim 4, wherein the scaffold is freeze-dried in a predetermined cast.

8. The scaffold according to claim 7, wherein the cast is prepared by (a) preparing a 3-dimensional (3D) skull mold using 3D computed tomography (CT) and (b) preparing a cast for preparation of the scaffold suitable for a bone defect area using a dental resin in the 3D skull mold.

9. The scaffold according to claim 4, wherein the bone powder is a ground bone powder from which osteoblasts are removed.

10. The scaffold according to claim 9, wherein the bone powder is derived from at least one selected from the group consisting of autogenous bone, allogeneic bone, xenogeneic bone, and synthetic bone.

11. The scaffold according to claim 5, wherein the fibrin glue comprises fibrinogen and thrombin.

12. The scaffold according to claim 11, wherein the fibrinogen is present in a concentration of 10 to 1000 mg/ml.

13. The scaffold according to claim 11, wherein the thrombin is present in a concentration of 0.1 to 1000 IU/ml.

14. The scaffold according to claim 5, wherein the fibrin glue further comprises aprotinin or calcium chloride.

15. The scaffold according to claim 5, wherein the fibrin glue further comprises a water-soluble binder.

16. The scaffold according to claim 15, wherein the water-soluble binder is a cell culture medium, distilled water, or blood.

17. The scaffold according to claim 5, wherein the bone powder and the fibrin glue are mixed in a volume ratio of 1 to 10:1.

18. The scaffold according to claim 5, wherein the bone growth promoting factor is a hormone, a cytokine, or a stem cell.


SEQUENCE LISTING

<110>  Wonkwang University Center for Industry-Academy Cooperation

<120>  BONE-REGENERATING COMPOSITION CONTAINING ANGIOGENIN

<130>  1115-PCT-EPO

<140>  PCT/KR2009/000500
<141>  2009-02-02

<150>  KR 10-2009-0003889
<151>  2009-01-16

<160>  1

<170>  PatentIn version 3.3

<210>  1
<211>  123
<212>  PRT
<213>  Artificial Sequence

<220>
<223>  angiogenin derived from bovine

<400>  1

Ala Gln Asp Asp Tyr Arg Tyr Ile His Phe Leu Thr Gln His Tyr Asp
1               5                   10                  15

Ala Lys Pro Lys Gly Arg Asn Asp Glu Tyr Cys Phe Asn Met Met Lys
            20                  25                  30

Asn Arg Arg Leu Thr Arg Pro Cys Lys Asp Arg Asn Thr Phe Ile His
        35                  40                  45

Gly Asn Lys Asn Asp Ile Lys Ala Ile Cys Glu Asp Arg Asn Gly Gln
    50                  55                  60

Pro Tyr Arg Gly Asp Leu Arg Ile Ser Lys Ser Glu Phe Gln Ile Thr
65                  70                  75                  80

Ile Cys Lys His Lys Gly Gly Ser Ser Arg Pro Pro Cys Arg Tyr Gly
                85                  90                  95

Ala Thr Glu Asp Ser Arg Val Val Gly Cys Glu Asn Gly Leu Pro Tyr
            100                 105                 110

His Phe Asp Glu Ser Phe Ile Thr Pro Arg His
        115                 120